(19) **Europäisches Patentamt European Patent Office Office européen des brevets**

(11) **EP 2 119 435 B1**

(12) **FASCICULE DE BREVET EUROPEEN**

(45) Date de publication et mention
de la délivrance du brevet:
**07.09.2011 Bulletin 2011/36**

(51) Int Cl.:
*A61K 31/01* (2006.01)　　　*A61K 31/047* (2006.01)
*A61P 17/02* (2006.01)　　　*A61P 17/06* (2006.01)
*A61K 8/30* (2006.01)　　　*A61K 8/92* (2006.01)

(21) Numéro de dépôt: **09290370.7**

(22) Date de dépôt: **18.05.2009**

(54) **Composition emolliente**

**Weichmachende Zusammensetzung**

**Emollient composition**

(84) Etats contractants désignés:
**AT BE BG CH CY CZ DE DK EE ES FI FR GB GR
HR HU IE IS IT LI LT LU LV MC MK MT NL NO PL
PT RO SE SI SK TR**

(30) Priorité: **16.05.2008 FR 0853187**

(43) Date de publication de la demande:
**18.11.2009 Bulletin 2009/47**

(73) Titulaire: **Pierre Fabre Dermo-Cosmétique
92100 Boulogne-Billancourt (FR)**

(72) Inventeurs:
• **Fabre, Pierre
81100 Castres (FR)**
• **Przybylski, Christophe
31290 Villefranche de Lauragais (FR)**
• **Cordoliani, Jean-François
31570 Sainte Foy D'Aigrefeuille (FR)**

• **Kopec, Marion
31400 Toulouse (FR)**

(74) Mandataire: **Warcoin, Jacques et al
Cabinet Regimbeau
20, rue de Chazelles
75847 Paris Cedex 17 (FR)**

(56) Documents cités:
**EP-A- 0 133 151　　WO-A-99/27904
CN-A- 1 552 303　　CN-A- 1 927 157
FR-A- 2 091 663　　PL-B3- 162 396**

• **KAMPF GÜNTER ET AL: "Regular use of a hand
cream can attenuate skin dryness and roughness
caused by frequent hand washing" BMC
DERMATOLOGY, BIOMED CENTRAL, LONDON,
GB, vol. 6, no. 1, 13 février 2006 (2006-02-13), page
1, XP021016988 ISSN: 1471-5945**

**Description**

L'invention concerne le domaine du traitement des pathologies et états associés à une fonction barrière de la peau altérée.

L'épiderme est un épithélium pluristratifié, d'origine ectodermique, en perpétuel renouvellement.

Il protège l'organisme de la déshydratation, des stress mécaniques et de certaines agressions pathogènes.

Il est composé de plusieurs types cellulaires : des kératinocytes à plus de 90% mais aussi des cellules de Langerhans, de Merkel et des mélanocytes.

On distingue plusieurs couches de nature morphologique et de composition cellulaire différentes, de l'intérieur vers l'extérieur : la couche basale, assise cellulaire dont les kératinocytes ont une capacité de prolifération très forte qui permet d'assurer l'auto-renouvellement de l'épiderme, puis les couches suprabasales (couches granuleuse, épineuse) et enfin la couche cornée (Stratum Corneum, SC).

Une des fonctions fondamentales de la peau est d'assurer une barrière entre l'organisme et le milieu extérieur s'opposant dans un sens à la pénétration dans l'épiderme de champignons, bactéries et allergènes de l'environnement et dans l'autre sens à la perte en eau.

La qualité de la fonction barrière est évaluée *in vivo* chez l'homme par la mesure de la perte insensible en eau et le taux d'hydratation et chez la souris par la mort par déshydratation des embryons, la perméabilité cutanée à un colorant ou la diminution de poids corporel.

L'intégrité du ciment lipidique extracellulaire ainsi que de tous les éléments cellulaires de la couche cornée et l'équilibre entre prolifération et différenciation kératinocytaires sont capitaux pour le maintien d'une fonction barrière épidermique fonctionnelle.

Le gradient de pH régule les activités des différentes enzymes et contribue ainsi à l'équilibre de la barrière.

En régulant la sécrétion des corps lamellaires dans la couche granuleuse, la concentration en ions calcium influence la composition du ciment extracellulaire de la couche cornée et ainsi l'équilibre de la barrière épidermique (Lee et al., Calcium and potassium are important regulators of barrier homeostasis in murine epidermis, J. Clin Invest, 89, 530-538, 1992).

La présence d'un gradient d'eau qui passe de 70% dans les couches visibles de l'épiderme à 30% dans les couches inférieures du SC et à 15% au niveau des couches de cellules les plus externes de l'épiderme (Warner et al, Electron probe analysis of human skin : determination of the water concentration profile, J. Invest Dermatol, 90, 218-224, 1988) suggère qu'une partie de l'eau est retenue dès la jonction entre couche granuleuse et couche cornée.

Une des fonctions de l'eau dans la couche cornée est de permettre les réactions d'hydrolyse enzymatique nécessaires à la flexibilité de la peau et à une desquamation normale. Si la quantité d'eau présente dans le SC descend en dessous d'un seuil critique, les réactions enzymatiques sont perturbées, conduisant à l'adhésion des cornéocytes et à l'accumulation des cellules à la surface de la peau. Cela crée une apparence visible de sécheresse, des démangeaisons, la peau pèle et s'écaille.

L'hydratation cutanée repose sur deux phénomènes : l'apport d'eau par le flux trans-épidermique apporté depuis la circulation sanguine et la rétention d'eau épidermique qui met en jeu la fonction barrière cutanée. Cependant la barrière vis-à-vis des pertes hydriques n'est pas absolue. Le mouvement normal d'échange d'eau entre le milieu externe et interne à travers la couche cornée est appelé TEWL (transepidermal water loss) et constitue une part de la perte insensible en eau.

La fonction barrière cutanée est altérée dans la majorité des pathologies de la peau les plus répandues dans la population et souvent accompagnées d'une composante inflammatoire (psoriasis, dermatite atopique, ichtyoses, sécheresse cutanée...). Elle l'est également dans un grand nombre d'états physiologiques en réponse au temps (vieillissement cutané) ou aux agressions de l'environnement (UVs, taux d'humidité, pollution, brûlures).

La perturbation de la fonction barrière, chronique ou aigue, rend l'organisme plus sensible aux agressions extérieures et à la déshydratation. Elle peut être associée à un dérèglement de la desquamation et à une hyperprolifération (Jackson et al. Pathobiology of the stratum corneum, West J. Med, 158, 279-285, 1993).

La demande FR2847467 concerne l'utilisation d'au moins un agent modulateur de l'activité de l'oxystérol $7\alpha$-hydroxylase pour la préparation d'une composition cosmétique destinée à prévenir et ou/traiter les désordres cutanés et/ou de membranes de type muqueuses affectant le bon fonctionnement de la barrière cutanée.

La demande FR2831443 concerne l'utilisation d'au moins un extrait de Gingko biloba ou d'Olea europaea, pour la préparation d'une composition destinée à améliorer la fonction barrière de la peau.

La demande FR2905857 concerne l'utilisation d'une composition comprenant un extrait de pulpe de caroube pour hydrater et/ou protéger de la sécheresse cutanée.

Des émulsions comprenant du glycérol, de la vaseline, de la paraffine liquide et de l'eau sont décrites dans le document Kampf et al., BMC Dermatology, 6 (1), 2006, 1471-5949 et dans le brevet FR 71.17627. Il existe un besoin en un traitement des pathologies et états associés à une fonction barrière de la peau altérée.

On a maintenant constaté de manière tout à fait surprenante et inattendue qu'une association de type glycérol, vaseline et paraffine liquide sous forme d'une émulsion huile dans eau ou eau dans huile permettait de traiter les états de sécheresse cutanée.

Les inventeurs ont mis en évidence que cette association restaure une barrière de la peau protectrice et fonctionnelle. Ils ont évalué l'activité hydratante de cette association et l'amélioration subséquente de la fonction barrière de la peau en utilisant un modèle de peau in vivo de déshydratation cutanée induite. En outre, ils ont observé l'expression des marqueurs épidermiques moléculaires potentiellement impliqués dans l'homéostasie de la fonction barrière épidermique par PCR quantitative et immuno-histochimie.

Les inventeurs ont également suivi l'activité serine protéase par zymographie in situ et la fonctionnalité de la barrière de la peau en utilisant des sondes fluorescentes. Les résultats montrent que l'association de type glycérol, vaseline et paraffine liquide sous forme d'une émulsion huile dans eau ou eau dans huile permet de restaurer l'activité sérine protéase et de supprimer l'inflammation induite par le stress.

Les inventeurs ont également mis en évidence que le choix d'une vaseline particulière dans cette association est particulièrement avantageuse pour atteindre les résultats ci-dessus. La vaseline en tant qu'agent occlusif et émollient est particulièrement importante dans la composition. EN effet, en formant un film protecteur sur la peau, elle permet d'aider à compenser la déficience de la fonction barrière altérée. La qualité du film formé sur la peau dépend très fortement des propriétés rhéologiques de la vaseline utilisée dans la fabrication.

La présente invention a ainsi pour objet une composition à usage topique comprenant, en tant que principe actif, une association de glycérol, vaseline et paraffine liquide, sous forme d'une émulsion huile dans eau ou eau dans huile.

Au sens de la présente invention, on appellera « association active » l'association glycérol, vaseline et paraffine liquide, sous forme d'une émulsion huile dans eau ou eau dans huile.

Avantageusement, le glycérol, la vaseline et la paraffine liquide présentent les critères décrits et contrôlés selon « European Pharmacopeia », 6ème Edition.

Avantageusement, la vaseline de l'association active présente un point de goutte compris entre 35 et 70°C, de préférence compris entre 51 et 57°C, de manière particulièrement préférée d'environ 54°C. Le point de goutte est mesuré selon le procédé 2.2.17 décrit dans « European Pharmacopeia », 6ème Edition.

Avantageusement, la vaseline de l'association active présente une consistance comprise entre 175 et 195 1/10 mm, de préférence d'environ 185 1/10 mm (pénétration au cône à 25°C).

Avantageusement, la vaseline de l'association active présente une viscosité comprise entre 4 et 5 cSt à 100°C, de préférence d'environ 4,8 cSt à 100°C.

Avantageusement, la vaseline de l'association active présente un spectre en spectroscopie RMN à 500 MHz du carbone C13 comprenant un pic à 24,55 ppm dont l'aire relative à un témoin de Tetra méthyl sylane (TMS) à 1% est comprise entre 4 et 8.

Dans la composition selon l'invention, l'association active est présente selon une proportion comprise entre 10 et 50 % et préférentiellement entre 20 et 30 % en poids par rapport au poids total de la composition ; la concentration en glycérol est comprise entre 5 et 30 %, préférentiellement entre 10 et 20 % et de manière particulièrement préférée est d'environ 15% en poids par rapport au poids total de la composition, la concentration en vaseline est comprise entre 3 et 20 %, préférentiellement entre 5 et 10 % et de manière particulièrement préférée est d'environ 8% en poids par rapport au poids total de la composition et la concentration en paraffine liquide est comprise entre 0,5 et 5 %, préférentiellement entre 1 et 3 % et de manière particulièrement préférée est d'environ 2% en poids par rapport au poids total de la composition.

Dans la phase aqueuse, l'eau est comprise entre 30 et 80 % en poids par rapport au poids total de la composition.

Avantageusement, la composition selon l'invention comprend environ 15% de glycérol, environ 8% de vaseline et environ 2% de paraffine liquide en poids par rapport au poids total de la composition.

La composition dermatologique selon l'invention comprend, en outre, des excipients usuels dermatologiquement compatibles.

La composition dermatologique selon la présente invention peut être préparée sous la forme d'une émulsion eau dans huile (E/H) ou huile dans eau (H/E), d'une émulsion multiple comme par exemple, une émulsion eau dans huile dans eau (E/H/E) ou une émulsion huile dans eau dans huile (H/E/H), ou encore sous la forme d'une hydrodispersion ou une lipodispersion, un gel ou un aérosol.

Les excipients dermatologiquement compatibles peuvent être tout excipient parmi ceux connus de l'homme de l'art en vue d'obtenir une composition pour l'application topique sous forme de crème, d'une lotion, d'un gel, d'une pommade, d'une émulsion, d'une microémulsion, d'un spray, etc.

La composition selon l'invention peut en particulier contenir des additifs et aides à la formulation, tels que des émulsionnants, des épaississants, des gélifiants, des fixateurs d'eau, des agents d'étalement, des stabilisants, des colorants, des parfums et des conservateurs.

Des émulsionnants appropriés comprennent l'acide stéarique, la trolamine, le PEG-40-stéarate.

De préférence, la composition selon l'invention présente environ 5% d'émulsionnants en poids par rapport au poids total de la composition.

Avantageusement, la composition selon l'invention présente entre 1 et 5% d'acide stéarique, de préférence environ 3% en poids par rapport au poids total de la composition.

Avantageusement, la composition selon l'invention présente entre 0 et 2% de trolamine, de préférence environ 0,5% en poids par rapport au poids total de la composition.

Avantageusement, la composition selon l'invention présente entre 0 et 2% de PEG-40-stéarate, de préférence environ 0,5% en poids par rapport au poids total de la composition.

Des épaississants appropriés comprennent le monostéarate de glycérol, le PEG 600.

De préférence, la composition selon l'invention présente environ 5% d'épaississants en poids par rapport au poids total de la composition.

Avantageusement, la composition selon l'invention présente entre 2 et 10% de monostéarate de glycérol, de préférence environ 5% en poids par rapport au poids total de la composition.

Avantageusement, la composition selon l'invention présente entre 2 et 10% de PEG 600, de préférence environ 5% en poids par rapport au poids total de la composition.

Des conservateurs appropriés comprennent le parahydroxybenzoate de propyle, le chlorocrésol.

De préférence, la composition selon l'invention présente environ 0,1% de conservateurs en poids par rapport au poids total de la composition.

Avantageusement, la composition selon l'invention présente entre 0,05 et 1% de parahydroxybenzoate de propyle, de préférence environ 0,1% en poids par rapport au poids total de la composition.

Des agents d'étalement appropriés comprennent la diméthicone, le polydiméthylcyclosiloxane.

De préférence, la composition selon l'invention présente environ 2% d'agents d'étalement en poids par rapport au poids total de la composition.

Avantageusement, la composition selon l'invention présente entre 0,2 et 2% de diméthicone, de préférence environ 0,5% en poids par rapport au poids total de la composition.

Avantageusement, la composition selon l'invention présente entre 1 et 3% de polydiméthylcyclosiloxane, de préférence environ 2,5% en poids par rapport au poids total de la composition.

Des fixateurs d'eau appropriés comprennent le polyéthylène glycol, de préférence le polyéthylène glycol 600.

De préférence, la composition selon l'invention présente environ 8% de fixateurs d'eau en poids par rapport au poids total de la composition.

Avantageusement, la composition selon l'invention présente entre 2 et 10% de polyéthylène glycol, de préférence environ 5% en poids par rapport au poids total de la composition.

L'eau utilisée pour la phase aqueuse de l'émulsion peut être une eau distillée ou une eau thermale ayant des propriétés dermato-cosmétique.

Avantageusement, la composition selon l'invention comprend :

- environ 15 % de glycérol,
- environ 8% de vaseline,
- environ 2% de paraffine liquide, et à titre d'excipients :
- environ 1 à 5% d'acide stéarique,
- environ 2 à 10% de monostéarate de glycérol,
- environ 1 à 3% de polydiméthylcyclosiloxane,
- environ 0,2 à 2% de diméthicone,
- environ 2 à 10% de polyéthylène glycol 600,
- environ 0 à 2% de trolamine,
- environ 0,05 à 1% de parahydroxybenzoate de propyle
- jusqu'à 100% en eau.

La présente invention a également pour objet l'utilisation d'une composition selon l'invention pour la préparation d'un médicament destiné à traiter les états de sécheresse cutanée, en particulier de certaines dermatoses telles que dermatite atopique, états ichtyosiques, psoriasis.

La présente invention a également pour objet l'utilisation d'une composition selon l'invention pour la préparation d'un médicament destiné à traiter les brûlures superficielles de faibles étendues.

La présente invention a également pour objet l'utilisation d'une composition selon l'invention pour la préparation d'un médicament destiné à prévenir et/ou traiter/réduire la fréquence et l'intensité des poussées eczémateuses observées chez les patients atteints de dermatite atopique.

La présente invention est illustrée par les exemples suivants.

## FIGURES

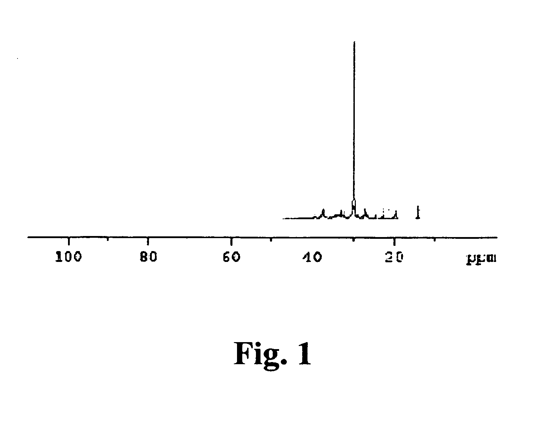

Figure 1 : Spectre RMN à 500 MHz du carbone C13 de 5 g d'échantillon de la vaseline Syntadex A (Synthéal) et de la Composition A.

## EXEMPLES

Exemple 1 : Formulations

Composition A

- 15 g de glycérol,
- 8 g de vaseline,
- 2 g de paraffine liquide,
- 0,5 g de trolamine,
- et à titre d'excipients acide stéarique, monostéarate de glycérol, polydiméthylcyclosiloxane, diméthicone, polyéthylène glycol (PEG) 600, parahydroxybenzoate de propyle
- eau jusqu'à 100 g.

Composition A'

- 15 g de glycérol,
- 8 g de vaseline,
- 2 g de paraffine liquide,
- 1,5 g d'acide stéarique,
- 5 g de monostéarate de glycérol,
- 1,5 g de polydiméthylcyclosiloxane,
- 0,5 g de diméthicone,
- 5 g de polyéthylène glycol 600,
- 0,15 g de trolamine,
- 0,1 g de parahydroxybenzoate de propyle
- eau jusqu'à 100 g.

Composition B

- 15 g de glycérol,
- 8 g de vaseline,
- 2 g de paraffine liquide,
- 0,5 g de PEG-40-stéarate,
- et à titre d'excipients acide stéarique, monostéarate de glycérol, polydiméthylcyclosiloxane, diméthicone, polyéthylène glycol 600, chlorocrésol,
- eau jusqu'à 100 g.

Composition B'

- 15 g de glycérol,
- 8 g de vaseline,
- 2 g de paraffine liquide,
- 3 g d'acide stéarique,
- 5 g de monostéarate de glycérol,

- **-** 2 g de polydiméthylcyclosiloxane,
- **-** 0,5 g de diméthicone,
- **-** 0,1 g de trolamine,
- **-** 3 g de polyéthylène glycol 600,
- **-** 0,5 g de PEG-40-stéarate,
- **-** 0,075 g de chlorocrésol,
- **-** eau jusqu'à 100 g.

<u>Exemple 2 : analyse de la régulation de la déshydratation cutanée induite</u>

Nous évaluons ici l'activité hydratante de la composition A et l'amélioration subséquente de la fonction barrière de la peau en utilisant un modèle de peau *ex vivo* de déshydratation cutanée induite.

Nous observons l'expression des marqueurs épidermiques moléculaires différentiels par PCR quantitative et immuno-histochimie.

Nous suivons également l'activité des enzymes à serine protéase par zymographie in situ et la dégradation de protéines coméodesmosomales par western blotting.

La fonctionnalité de la barrière de la peau est analysée en utilisant des sondes fluorescentes.

**Matériel et méthodes**

**I. Modèles tissulaires**

**1. Préparation des explants cutanés**

Le laboratoire récupère des prélèvements de peau provenant de déchets opératoires de chirurgie plastique (diminutions mammaires). L'utilisation de ces prélèvements rentre dans le cadre de « la déclaration d'activité de conservation et de préparation d'éléments du corps humain pour les besoins de programme de recherche du groupe Pierre Fabre » faite au Ministère de l'Enseignement Supérieur et de la Recherche.

Ces prélèvements sont lavés dans 10 bains de PBS puis découpés à l'emporte-pièce en disques de 2 cm de diamètre. Les explants cutanés sont étalés sur une grille dans une boîte de Pétri et un anneau de 1 cm de diamètre est scellé sur la peau pour délimiter la zone de traitement.

**2. Cinétique des modèles**

Pour le modèle de déshydratation induite, la peau est desséchée pendant 2 heures sous la hotte de culture cellulaire dans une boîte sans couvercle puis est mise à l'étuve pour un traitement topique avec ou sans association active pendant 2 heures. Le témoin négatif du stress de déshydratation subit la même cinétique dans une boîte de Pétri fermée.

**3. Prélèvements pour l'analyse**

Après le traitement, 2 biopsies de 6 mm de diamètre sont prélevées pour l'analyse de l'expression des ARN et une biopsie de 4 mm de diamètre incluse dans un bloc de résine Tissue Tek® (Sakura Finetek) pour l'histologie. Pour l'analyse des protéines, la peau est exposée à un choc thermique dans un bain d'eau à 60°C pendant 5 minutes puis à 4°C pendant 2 minutes afin de séparer l'épiderme du derme.

Les biopsies et les épidermes sont congelés dans l'azote liquide et stockés à -80°C avant d'être analysés.

**II. Analyse du transcriptome par PCR quantitative**

Les biopsies de peau sont broyées dans un mortier préalablement refroidi à l'azote liquide et les ARNs sont extraits grâce à un kit RNeasy® (QIAGEN) selon les recommandations du fournisseur. L'ARN est ensuite dosé à l'aide d'un Bioanalyser 2100® (Agilent Technologies) sur puces RNA 6000 Nano LabChip®. L'ADNc est obtenu à partir d'l $\mu$g d'ARN par réaction enzymatique de rétro-transcription réalisée avec un kit Acces RT-PCR Core Reagents® (Promega), à l'aide d'amorces oligo dT. Les niveaux d'expression génique sont analysés par PCR quantitative sur un thermocycleur à fluorescence ICycler iQ® (Biorad) avec des kits PCR iQ™SYBR® Green Super Mix (Biorad) suivant un protocole de 40 cycles comprenant une dénaturation à 95°C (15 sec) et une élongation à 60°C (1 mm). L'accumulation du produit de PCR proportionnelle à l'émission de fluorescence (intercalant SYBR®Green) est visualisée cycle après cycle grâce au logiciel iCycler.

Le logiciel d'analyse iCycler version 3.1 délivre des valeurs brutes de $C_T$ (Cycle Threshold): cycle à partir duquel l'amplification d'ADNc débute. L'expression de plusieurs gènes de référence est analysée en parallèle à l'aide du programme Genorm version 3.4 qui permet de choisir le gène de référence le plus stable d'un échantillon à l'autre. Ce gène sert ensuite de référence pour la normalisation des résultats par le calcul du $\Delta C_T = C_T$ gène d'intérêt - $C_T$ gène de référence.

Le facteur d'induction (FI) est ensuite calculé pour chaque traitement par rapport à la condition contrôle correspondante. FI = $2^{-\Delta\Delta Cr}$ où $\Delta\Delta C_T = \Delta CT$ traité - $\Delta CT$ contrôle L'expression des ARNm est évaluée en duplicat pour cinq expériences provenant de 5 individus différents. Lorsque le facteur d'induction par rapport au contrôle est supérieur à 2, on considère que l'expression du gène est induite et lorsqu'il est inférieur à 0,5, on considère que l'expression est réprimée. L'effet du principe actif sur la réponse au stress causée dans le modèle est évalué par le pourcentage d'inhibition calculé avec la fonale suivante:

$$\% \text{ d'inhibition} = 100 * \frac{(\text{FI stressé} - \text{FI témoin sans stress}) - (\text{FI traité} - \text{FI témoin sans stress})}{(\text{FI stressé} - \text{FI ténsoin sans stress})}$$
de réponse au stress

Par rapport au modèle d'étude, la condition « témoin sans stress » correspond au contrôle non desséché; la condition «stressé» correspond à une biopsie de peau qui a été desséchée 2 heures puis qui a passé 2 heures supplémentaire en condition contrôle (c'est-à-dire sans traitement topique); enfin la condition «traité» est la peau qui a subi 2 heures de dessèchement suivies de 2 heures de traitement topique par un émollient.

### III. Analyse de l'expression protéique par Western Blot

Les épidermes traités sont broyés dans un mortier refroidi à l'azote liquide et les protéines sont extraites dans un tampon de lyse RIPA (Tris HCl pH8 5OmM; NaCL 15OmM; Triton X 100 IX; Na+Desoxycholate 1%; SDS 0,1%; EDTA 5mM ; DTT 100mM; cocktail d'inhibiteur de protéases (référence P8340, SIGMA).

Les protéines sont ensuite dosées par la méthode DC-DC Protein Assay (Biorad) et analysées par Western Blot. Pour chaque condition, 25 à 40$\mu$g de protéines totales sont déposées sur des gels Tris-Glycine à 7,5% de polyacrylamide. Le mélange protéique est séparé par électrophorèse à l'aide du système Mini Protean II (Biorad) et les protéines sont transférées sur une membrane de PVDF (Hybond-P, Amersham). La protéine d'intérêt est révélée par un anticorps spécifique et un kit ECL+ (Amersham). La quantité de protéines et la proportion de forme dégradée sont calculées grâce au logiciel Image Master TotalLab version 1.11 (Amersham) après normalisation par rapport à la $\beta$-actine (protéine de référence).

### IV. Techniques histologiques

Les biopsies de peau sont sectionnées au cryotome (Leica CM 3050s) en coupes de 5$\mu$m d'épaisseur et déposées sur des lames d'observation (Starfrost®).

### 1. Immunohistochimie

Les cryocoupes sont fixées 10 minutes à l'acétone à 20°C puis réhydratées au PBS avant d'être analysées par marquage immunochimique. Après fixation et réhydratation, les coupes de peau sont saturées avec une solution de BSA 3% et incubées 1 heure avec l'anticorps primaire dirigé contre la protéine d'intérêt. Dans un deuxième temps, elles sont incubées pendant 1 heure avec l'anticorps secondaire couplé à un fluorochrome Alexa-488 ou Alexa-555 et enfin montées dans du Mowiol contenant du DAPI pour marquer les noyaux.

### 2 Zymographie *in situ*

Après une fixation de 10 minutes dans l'acétone à -20°C, les coupes sont rincées dans une solution de lavage (Tween 20 1% dans l'eau) et incubées 2 heures à 37°C avec une solution contenant le substrat spécifique des enzymes d'intérêt couplé à un fluorophore (annexe). Lorsque l'enzyme est active, le fluorophore est clivé, libérant un signal fluorescent observable au microscope. Les lames marquées sont ensuite observées au microscope à épifluorescence (Nikon Eclipse *50i*) ou au microscope confocal inversé Zeiss Axiovert 100.

**3 Sonde fluorescente**

Après le traitement de déshydratation, les explants cutanés sont incubés pendant une heure supplémentaire à l'étuve à 37°C avec une sonde fluorescente Lucifer Yellow Carboxyhydrazide dilithium salt (Invitrogen) à 1mM dans du tampon HBSS. La peau est ensuite rincée dans un bain d'HBSS pendant 1 minute puis des biopsies de 4mm de diamètre sont prélevées et incluses dans de la résine Tissue TekR® (Sakura Finetek) (Matsuki et al 1998). La peau est ensuite coupée, les noyaux sont marqués au DAPI et les lames sont observées au microscope à fluorescence à une longueur d'onde de 450 nm comme décrit ci-dessus.

**Résultats**

**1. Modèle de rupture de la fonction barrière par dessèchement induit**

**1. Mesure de la perméabilité cutanée par une sonde fluorescente**

La première analyse a consisté à étudier un paramètre fonctionnel fondamental dans la fonction barrière cutanée : la perméabilité des couches supérieures de l'épiderme. L'incubation de la peau avec une sonde fluorescente (Lucifer Yellow) après l'expérience de dessèchement a permis de caractériser la modulation de la perméabilité cutanée. En condition contrôle, le marquage est très faible et superficiel, la sonde pénètre peu à travers la couche cornée et est éliminée lors du rinçage. Après deux heures de dessèchement, le marquage est observable dans les strates plus profondes de la couche cornée. Le dessèchement rend la peau plus perméable, sa fonction barrière est détériorée. Le traitement topique par la composition A suite aux deux heures de dessèchement restaure l'imperméabilité du SC vis à vis de la sonde, le marquage est à nouveau faible et superficiel, comme dans la condition contrôle. On peut alors conclure que le traitement hydratant a un effet réparateur sur la peau desséchée et sur la fonction barrière cutanée observable sur le modèle tissulaire.

**2. Effets sur la régulation du transcriptome et du protéome**

L'expression de différents gènes potentiellement impliqués dans l'homéostasie de la fonction barrière épidermique a été mesurée par PCR quantitative dans les différentes conditions de stress ou de traitement du modèle de dessèchement. L'analyse par immunohistochimie a permis de montrer la réorganisation de l'expression de certaines protéines en terme de localisation, par exemple les jonctions serrées. La dégradation des protéines cornéodesmosomales a été analysée par Western-Blot.

Les cibles étudiées à l'aide de ces différentes approches ont été regroupées selon leur rôle physiologique (voir tableau 1). L'objectif de cette étude est d'observer une réponse au stress visualisable et une correction de l'effet du stress par l'application topique de la composition A.

Le travail réalisé a également permis de mettre en évidence les différents niveaux de régulation de certaines cibles. Nous avons ainsi pu constater que les enzymes de la desquamation n'étaient pas régulées au niveau transcriptionnel mais plus spécialement au niveau de leur activité (cf. Résultats 3).

**Tableau 1 : Récapitulatif des cibles et de la réponse pharmacologique étudiées dans le modèle de dessèchement.**

| O=La cible réagit dans le modèle ;<br>X=La cible ne réagit pas dans le modèle | | O=Oui<br>N=Non |
|---|---|---|
| Outils | Réponse au stress | Inhibition de la réponse au stress par la composition A |
| Perméabilité cutanée | O | O |
| Desmogléine 1 | O | O |
| Cdsn | X | X |
| Desmocolline | O | O |
| Plakoglobine | O | X |
| KLK5 | O | X |
| KLK7 | X | X |

(suite)

| O=La cible réagit dans le modèle ;<br>X=La cible ne réagit pas dans le modèle | | O=Oui<br>N=Non |
| --- | --- | --- |
| Outils | Réponse au stress | Inhibition de la réponse au stress par la composition A |
| KLK8 | X | X |
| Protéases à sérine | O | O |
| Cathépsine D | X | X |
| ABC A12 | O | O |
| ABC G1 | X | X |
| B-GC | X | X |
| DES 2 | X | X |
| Filaggrine | O | O |
| Involucrine | X | X |
| TG1 | X | X |
| TG3 | X | X |
| Caspase 14 | X | X |
| Elox-3 | O | X |
| 12R-LOX | X | X |
| HAS 2 | O | X |
| CD44 | X | X |
| AQP3 | O | O |
| NHE1 | O | O |
| IL-1a | O | O |
| Occludine | O | O |
| Claudine 1 | O | O |
| Claudine 4 | O | O |
| ZO-1 | X | X |
| E-Cadherine | X | X |
| βCatenine | X | X |

### 3. Mesure de l'activité enzymatique liée à la desquamation

L'activité des protéases à sérine a été évaluée par zymographie *in Situ* sur le modèle de déshydratation et observée au microscope confocal dans la condition contrôle après deux heures de dessèchement et après deux heures de dessèchement suivies par une incubation de deux heures avec la composition A. Le marquage est le plus intense dans la condition contrôle, il correspond à l'activité forte normale. Ce marquage diminue et devient irrégulier le long de la couche cornée après deux heures de dessèchement alors que son intensité est augmentée et la localisation de l'activité réorganisée après les deux heures d'incubation avec la composition A. Le dessèchement a pour effet de diminuer et perturber l'activité enzymatique. Ces résultats sont cohérents avec la diminution de la dégradation des protéines coméodesmosomales que nous observons avec le dessèchement et confirme l'effet du dessèchement sur la diminution de la desquamation observée sur le modèle mis au point. La composition A est capable de restaurer l'activité enzymatique de la peau déshydratée ce qui confirme l'effet de cette composition pour un retour de l'homéostasie de la desquamation.

Ces résultats montrent que la composition A permet de restaurer le niveau d'expression des cibles moléculaires dont l'expression est accrue par le stress de déshydratation cutané induit. La composition A permet également de restaurer l'activité sérine protéase. En outre, l'application topique de la composition A permet de supprimer l'inflammation induite par le stress.

L'ensemble de ces résultats suggère que la composition A en application topique permet de restaurer la fonction barrière de la peau, de limiter

Exemple 3 : Caractérisation par RMN de la vaseline Syntadex A

5 g d'échantillon sont solubilisés dans du chloroforme deutéré pour mesure par RMN à 500 MHz du carbone C13.

La vaseline Syntadex A (Synthéal) présente un spectre caractéristique en spectroscopie RMN à 500 MHz du carbone C13, comprenant notamment un pic à 24,55 ppm dont l'aire relative à un témoin de Tetra méthyl sylane (TMS) à 1% est comprise entre 4 et 8.

Ce même pic se retrouve dans la Composition A.

**Revendications**

1. Composition à usage topique comprenant, en tant que principe actif, une association de glycérol, vaseline et paraffine liquide sous forme d'une émulsion huile dans eau ou eau dans huile dans laquelle la vaseline présente un point de goutte compris entre 51 et 57°C, une consistance comprise entre 175 et 195 1/10 mm (pénétration au cône à 25°C) et une viscosité comprise entre 4 et 5 cSt à 100°C.

2. Composition selon la revendication 1 dans laquelle la vaseline présente un spectre en spectroscopie RMN à 500 MHz du carbone C13 comprenant un pic à 24,55 ppm dont l'aire relative à un témoin de Tetra méthyl sylane (TMS) à 1% est comprise entre 4 et 8.

3. Composition selon la revendication 1 ou 2 dans laquelle la vaseline présente un point de goutte de 54°C.

4. Composition selon l'une quelconque des revendications précédentes dans laquelle la vaseline présente une consistance d'environ 185 1/10 mm (pénétration au cône à 25°C).

5. Composition selon l'une quelconque des revendications précédentes dans laquelle la vaseline présente une viscosité d'environ 4,8 cSt à 100°C.

6. Composition selon l'une quelconque des revendications précédentes comprenant environ 15% de glycérol, environ 8% de vaseline et environ 2% de paraffine liquide.

7. Composition selon l'une quelconque des revendications précédentes comprenant un ou plusieurs excipients choisis dans le groupe constitué de acide stéarique, monostéarate de glycérol, polydiméthylcyclosiloxane, diméthicone, polyéthylène glycol 600, trolamine, parahydroxybenzoate de propyle, chlorocrésol, PEG-40-stéarate, eau distillée.

8. Utilisation d'une composition selon l'une quelconque des revendications précédentes pour la préparation d'un médicament destiné à traiter les états de sécheresse cutanée de certaines dermatoses telles que dermatite atopique, états ichtyosiques, psoriasis.

9. Utilisation d'une composition selon l'une quelconque des revendications 1 à 7 pour la préparation d'un médicament destiné à traiter les brûlures superficielles de faibles étendues.

10. Utilisation d'une composition selon l'une quelconque des revendications 1 à 7 pour la préparation d'un médicament destiné à prévenir et/ou traiter/réduire la fréquence et l'intensité des poussées eczémateuses observées chez les patients atteints de dermatite atopique.

11. Utilisation d'une vaseline présentant un point de goutte compris entre 51 et 57°C pour préparer une composition à usage topique comprenant, en tant que principe actif, une association de glycérol, vaseline et paraffine liquide sous forme d'une émulsion huile dans eau ou eau dans huile.

12. Utilisation d'une vaseline présentant un spectre en spectroscopie RMN à 500 MHz du carbone C13 comprenant un pic à 24,55 ppm dont l'aire relative à un témoin de Tetra méthyl sylane (TMS) à 1% est comprise entre 4 et 8 pour préparer une composition à usage topique comprenant, en tant que principe actif, une association de glycérol, vaseline et paraffine liquide sous forme d'une émulsion huile dans eau ou eau dans huile.

**Claims**

1. A composition for topical use that comprises a combination of glycerol, vaseline and liquid paraffin as an active ingredient, in the form of an oil-in-water or water-in-oil emulsion in which the vaseline has a drop point between 51°C and 57°C, a consistency between 175 1/10 mm and 195 1/10 mm (cone penetration at 25°C) and a viscosity between 4 cSt and 5 cSt at 100°C.

2. The composition according to claim 1 in which the vaseline has a carbon-13 NMR spectroscopy spectrum at 500 MHz that comprises a peak at 24.55 ppm whose area relative to a 1% tetramethylsilane (TMS) control is between 4 and 8.

3. The composition according to claim 1 or 2 in which the vaseline has a drop point of 54°C.

4. The composition according to any of the preceding claims in which the vaseline has a consistency of approximately 185 1/10 mm (cone penetration at 25°C).

5. The composition according to any of the preceding claims in which the vaseline has a viscosity of approximately 4.8 cSt at 100°C.

6. The composition according to any of the preceding claims that comprises approximately 15% glycerol, approximately 8% vaseline and approximately 2% liquid paraffin.

7. The composition according to any of the preceding claims that comprises one or more excipients selected from the group comprised of stearic acid, glycerol monostearate, polydimethylcyclosiloxane, dimethicone, polyethylene glycol 600, trolamine, propyl parahydroxybenzoate, chlorocresol, PEG-40-stearate, distilled water.

8. Use of the composition according to any of the preceding claims for preparing a drug for treating the conditions of cutaneous dryness of certain dermatoses such as atopic dermatitis, ichthyosis conditions and psoriasis.

9. Use of the composition according to any of claims 1 to 7 for preparing a drug for treating small superficial burns.

10. Use of the composition according to any of claims 1 to 7 for preparing a drug for preventing and/or treating and/or reducing the frequency and the intensity of the eczema attacks observed among patients suffering from atopic dermatitis.

11. Use of a vaseline with a drop point between 51°C and 57°C for preparing the composition for topical use that comprises a combination of glycerol, vaseline and liquid paraffin as an active ingredient, in the form of an oil-in-water or water-in-oil emulsion.

12. Use of a vaseline with a carbon-13 NMR spectroscopy spectrum at 500 MHz that comprises a peak at 24.55 ppm whose area relative to a 1% tetramethylsilane (TMS) control is between 4 and 8 for preparing the composition for topical use that comprises a combination of glycerol, vaseline and liquid paraffin as an active ingredient, in the form of an oil-in-water or water-in-oil emulsion.

**Patentansprüche**

1. Zusammensetzung zur topischen Anwendung, umfassend als Wirkstoff eine Assoziation von Glycerol, Vaseline und flüssigem Paraffin in Form einer Öl-in-Wasser- oder Wasser-in-Öl-Emulsion, wobei die Vaseline einen Tropfpunkt von 51 bis 57 °C, eine Konsistenz von 175 bis 195 1/10 mm (Penetration in den Konus bei 25 °C) und eine Viskosität von 4 bis 5 cSt bei 100 °C aufweist.

2. Zusammensetzung gemäß Anspruch 1, wobei die Vaseline ein Spektrum in der Kohlenstoff-C13-NMR-Spektroskopie bei 500 MHz aufweist, welches einen Peak bei 24,55 ppm umfasst, dessen Flächeninhalt im Verhältnis zu einem Tetramethylsilan (TMS)-Vergleich (1 %) 4 bis 8 beträgt.

3. Zusammensetzung gemäß Anspruch 1 oder 2, wobei die Vaseline einen Tropfpunkt von 54 °C aufweist.

4. Zusammensetzung gemäß irgendeinem der vorhergehenden Ansprüche, wobei die Vaseline eine Konsistenz von ungefähr 185 1/10 mm (Penetration in den Konus bei 25 °C) aufweist.

5. Zusammensetzung gemäß irgendeinem der vorhergehenden Ansprüche, wobei die Vaseline eine Viskosität von ungefähr 4,8 cSt bei 100 °C aufweist.

6. Zusammensetzung gemäß irgendeinem der vorhergehenden Ansprüche, welche ungefähr 15 % Glycerol, ungefähr 8 % Vaseline und ungefähr 2 % flüssiges Paraffin enthält.

7. Zusammensetzung gemäß irgendeinem der vorhergehenden Ansprüche, welche einen oder mehrere Zusatzstoffe, ausgewählt aus der Gruppe bestehend aus Stearinsäure, Glycerolmonostearat, Polydimethylcyclosiloxan, Dimethicon, Polyethylenglycol 600, Trolamin, Propylparahydroxybenzoat, Chlorchresol, PEG-40-Stearat, destilliertes Wasser, enthält.

8. Verwendung einer Zusammensetzung gemäß irgendeinem der vorhergehenden Ansprüche zur Herstellung eines Medikaments zur Behandlung von Zuständen der Hauttrockenheit bei bestimmten Dermatosen, wie beispielsweise atopische Dermatitis, ichtyosische Zustände, Psoriasis.

9. Verwendung einer Zusammensetzung gemäß irgendeinem der Ansprüche 1 bis 7 zur Herstellung eines Medikaments zur Behandlung von oberflächlichen Verbrennungen schwacher Ausprägung.

10. Verwendung einer Zusammensetzung gemäß irgendeinem der Ansprüche 1 bis 7 zur Herstellung eines Medikaments zur Vorbeugung und/oder Behandlung/Reduzierung der Häufigkeit und der Intensität von ekzematösen Hautausschlägen, die bei Patienten mit atopischer Dermatitis beobachtet werden.

11. Verwendung einer Vaseline, welche einen Tropfpunkt von 51 bis 57 °C aufweist, zur Herstellung einer Zusammensetzung zur topischen Anwendung, welche als Wirkstoff eine Assoziation von Glycerol, Vaseline und flüssigem Paraffin in Form einer Öl-in-Wasser- oder Wasser-in-Öl-Emulsion umfasst.

12. Verwendung einer Vaseline, welche ein Spektrum in der Kohlenstoff-C13-NMR-Spektroskopie bei 500 MHz aufweist, welches einen Peak bei 24,55 ppm umfasst, dessen Flächeninhalt im Verhältnis zu einem Tetramethylsilan (TMS)-Vergleich (1 %) 4 bis 8 beträgt, zur Herstellung einer Zusammensetzung zur topischen Anwendung, welche als Wirkstoff eine Assoziation von Glycerol, Vaseline und flüssigem Paraffin in Form einer Öl-in-Wasser- oder Wasser-in-Öl-Emulsion umfasst.

**Fig. 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2847467
- FR 2831443
- FR 2905857
- FR 7117627

**Littérature non-brevet citée dans la description**

- **Lee et al.** Calcium and potassium are important regulators of barrier homeostasis in murine epidermis. *J. Clin Invest,* 1992, vol. 89, 530-538
- **Warner et al.** Electron probe analysis of human skin : determination of the water concentration profile. *J. Invest Dermatol,* 1988, vol. 90, 218-224
- **Jackson et al.** Pathobiology of the stratum corneum. *West J. Med,* 1993, vol. 158, 279-285
- **Kampf et al.** *BMC Dermatology,* 2006, vol. 6 (1), 1471-5949